# EUROPEAN PATENT SPECIFICATION

(11) **EP 1 402 875 B1**
(45) Date of publication and mention of the grant of the patent: **05.10.2011**
(21) Application number: 02741417.6
(22) Date of filing: 05.07.2002
(51) Int. Cl.: A61K 8/11, A61K 8/58, A61K 8/69, A61K 8/70, A61K 8/92, A61Q 1/12, C09D 5/03, A61K 8/897

(54) **POWDERY COMPOSITION**
PUDERZUSAMMENSETZUNG
COMPOSITION EN POUDRE

(30) Priority: 05.07.2001 JP 2001204362; 05.07.2001 JP 2001204363; 05.07.2001 JP 2001204364; 20.03.2002 JP 2002078689
(43) Date of publication of application: 31.03.2004
(73) Proprietor: SHISEIDO COMPANY, LTD., Tokyo 104-8010 (JP)
(72) Inventor: SATO, Tomoko, Shiseido Res. Center (Shin-Yokohama), Yokohama-shi, Kanagawa 224-8558 (JP); KANEMARU, T., Shiseido Res. Center (Shin-Yokohama), Yokohama-shi, Kanagawa 224-8558 (JP); MATSUZAKI, F., Shiseido Res. Center(Shin-Yokohama), Yokohama-shi, Kanagawa 224-8558 (JP); YANAKI, T., Shiseido Res. Center (Shin-Yokohama), Yokohama-shi, Kanagawa 224-8558 (JP)
(74) Representative: Quantin, Bruno Marie Henri
(86) International application number: PCT/JP2002/006859
(87) International publication number: WO 2003/003990

(56) References cited:
- EP-A2- 0 855 177
- WO-A1-02/56844
- JP-A- 4 009 323
- JP-A- 5 065 212
- JP-A- 6 211 620
- JP-A- 11 130 614
- JP-A- 2000 247 823
- JP-A- 2000 256 583
- JP-A- 2000 264 813
- JP-A- 2000 309 505
- JP-A- 2000 309 506
- JP-A- 2001 158 716
- US-A- 5 578 311
- CHEMICAL ABSTRACTS, vol. 121, no. 16, 17 October 1994 (1994-10-17), Columbus, Ohio, US; abstract no.: 186793, XP002388921 & JP 06 166611 A (KOSE CORP) 14 June 1994 (1994-06-14)
- PATENT ABSTRACTS OF JAPAN vol. 011, no. 232 (C-437), 29 July 1987 (1987-07-29) & JP 62 045666 A (POLA CHEM IND INC), 27 February 1987 (1987-02-27)

## Description

### TECHNICAL FIELD

The present invention relates to a powder composition (i.e., dry oil) which is composed of an oily component in the form of an oil drop shape homogeneously covered on its surface with a fluorine-treated powder and which, while a powder in form, liquifies by friction of an application thereof at the time of use. This powder composition is used in the field of cosmetics as a powder cosmetic composition and is used in the field of ink and printing as a powder type oil paint, etc. Particularly, it relates to a new type of powder cosmetic composition which, as a powder cosmetic composition, is free of a sticky feeling, can suppress chapping of the skin, can effectively prevent adhesion of oil, dirt, etc. on the container in spite of an oil-rich preparation, and is imparted with a fresh feeling. The present invention further relates to a new type of powder cosmetic composition which does not contain substantially any surfactant, preservative, water, etc. or even if containing them, without substantially containing water requires only a small amount, is free from a sticky feeling, can prevent chapping of the skin, can effectively prevent adhesion of oil, dirt, etc. on the container in spite of an oil-rich preparation, can stably include even unstable ingredients which easily break down in the presence of light, oxygen, water, etc. and easily give rise to odor, discoloration, etc., and can sufficiently exhibit the functions of such ingredients.

### BACKGROUND ART

In recent years, in the cosmetic industry, consumers are becoming increasingly interested in safety. Cosmetics free of preservatives and other additives are becoming popular. As one type of such cosmetics, products containing, for example, oils such as virgin olive oil, lavender oil, as they are for direct application to the skin are being marketed. Such products are highly effective in suppressing chapping of the skin, but usually feel sticky or slimy and cannot be said to be preparations with a good feeling of use. Further, the containers containing such oil-rich preparations have been disadvantageous in that the oil would adhere to the outside of the containers during use and, along with this, a remarkable extent of dirt would adhere to the containers. Further, the diversification in consumer preferences has led to a demand for even oil-rich preparations giving new feelings such as a fresh feeling.

US 5 578 311 discloses cosmetics containing a hydrophobic powder or a fluorine compound-treated powder, and a liquid perfluoro organic compound.

JP-A-6 166 611 discloses a powdery cosmetic composition comprising 0.1-7wt% particules of hydrophobized silic anhydride, 1-50 wt% of cosmetic powder coated with a fluorine compound, 30-40wt% of an oily ingredient and 30-90wt% of an aqueous ingredient.

A powdery cosmetic composition comprising 0.1-7wt% of a hydrophobic silicic anhydride coated with an organosilane-based compound or a fluorine compound, 1-50wt% of cosmetic powder coated with a fluorine compound, 0.5-40wt% of an oily ingredient, 30-90wt% of an aqueous ingredient and 0.001-10wt% of an unstable active component is disclosed in JP-A-6 211 620.

JP-A-5 065 212 relates to an aqueous powder composition obtained by mixing hydrophobic silica, water, a cosmetic powder treated with a fluorine compound, and an oily ingredient.

Further, in general, cosmetics do not contain surfactants and include various active ingredients such as medicines. When these active ingredients are unstable, e.g., would easily break down and spoil in the presence of light, oxygen, water, etc., and have a detrimental effect on the physical properties of the product, inclusion is restricted not only by the form of the cosmetic, but also the type of the container, storage conditions, handling, etc. Therefore, preparations which can stably include such unstable ingredients have been desired.

### DISCLOSURE OF INVENTION

The present invention was made in consideration of the above situation and the object thereof is to provide a powder composition which does not have to contain a preservative or other additive, which despite being an oil-rich preparation highly effective in suppressing chapping of the skin, is a cosmetic superior in feeling of use with no stickiness at the time of application to the skin and is suitable for use as an easy-to-use powder cosmetic composition free from adhesion of oil to the storage container even with continuous long-term use.

Another object of the present invention is to provide a powder cosmetic composition which, in spite of an oil-rich preparation highly effective in suppressing chapping of the skin, imparts a fresh feeling and is superior in feeling of use with no stickiness at the time of application to the skin, and is easy to use being free from adhesion of oil to the storage container even with continuous long-term use.

A further object of the present invention is to provide a powder cosmetic composition which does not contain a surfactant, preservative, water, etc. or even if containing them, requires only a small amount, which, in spite of an oil-rich preparation highly effective in suppressing chapping of the skin, is a cosmetic composition superior in feeling of use with no stickiness at the time of application to the skin, is easy to use without adhesion of oil to the storage container even with continuous long-term use, and which can stably include even unstable ingredients which easily break down, spoil, etc. in the presence of light, oxygen, water, etc. and can sufficiently exhibit the functions of such ingredients.

In accordance with the present invention, there is provided a powder composition comprising (a) a fluorine-treated powder and (b) an oily component having a surface tension value (average) of at least 2.0 x 10⁻² N/m, without substantially containing water, which is capable of liquifying by the friction of an application thereof.

In accordance with the present invention, there is also provided the above powder composition, wherein the component (a) is a fluorine-treated powder of a clay mineral treated on its surface with fluorine.

In accordance with the present invention, there is further provided the above powder composition, wherein the component (a) is a fluorine-treated powder of a clay mineral treated on its surface with at least one gel selected from the group consisting of hydrates, partial dehydrates, and anhydrides of metal hydroxides or metal salts, followed by being treated with fluorine.

In accordance with the present invention, there is further provided the above powder composition, wherein the clay mineral is at least one mineral selected from the group consisting of talc, kaolin, silicic anhydride, saponite, sericite, mica, and boron nitride.

In accordance with the present invention, there is further provided the above powder composition, wherein the ratio of the component (a) and the component (b) is 1:9 to 4:1 (ratio by weight).

The powder composition according to the present invention can be used as a powder cosmetic composition or a powder paint.

In accordance with a third aspect of the present invention, there is provided a powder cosmetic composition comprising (a) a fluorine-treated powder, (b) an oily component having a surface tension value (average) of at least 2.0 x 10⁻² N/m, and (c) a component unstable in the presence of light, oxygen or water, which is capable of liquifying by friction of an application thereof.

In accordance with the present invention, there is also provided the above powder cosmetic composition, wherein the component (a) is a fluorine-treated powder of a clay mineral treated on its surface with fluorine.

In accordance with the present invention, there is further provided the above powder cosmetic composition, wherein the component (c) is at least one member selected from the group consisting of whitener, anti-inflammatory agent, humectant, blood circulation promoter, hair tonic, antibacterial agent, hormone, vitamin, enzyme, antioxidant, and animal or plant extract.

In accordance with the present invention, there is further provided the above powder cosmetic composition containing the component (c) in an amount of 0.001 to 10% by weight.

### MODE FOR CARRYING OUT THE INVENTION

Next, the present invention will be explained in detail. Note that in the present specification and the claims, singular forms of expression shall be deemed to mean either singular or plural forms except when otherwise clear from the context.

The invention relates to a powder cosmetic composition comprising
(a) a fluorine-treated powder and
(b) an oily component,
without substantially containing water,
wherein,
component (a) is present in an amount of 20 to 70% by weight based upon the total amount of the composition,
the oily component (b) has a surface tension value (average) with respect to the atmosphere of at least 2.0 x 10⁻² N/m,
the ratio of the component (a) and component (b) is 1:9 to 4:1 (ratio by weight)
and
the oily component (b) is powderized by absorbing component (a),
which composition is capable of liquifying by friction on application thereof.

The fluorine-treated powder usable as the component (a) in the present invention includes broadly powder given fluorine groups on its surface to impart water repellency and oil repellency and powder having fluorine groups itself and thereby having water repellency and oil repellency. Specifically, (i) treated powder treated on part or all of the powder surface with molecules having fluorine groups and (ii) polymer powder including fluorine groups etc. may be mentioned, but the present invention is not limited to these examples.

In the above (i), the molecules having fluorine groups are not particularly limited, but fluorine compounds such as a perfluoroalkyl phosphoric acid ester diethanolamine salt expressed by the following formula (I), a perfluoroalkylsilane expressed by the following formula (II), a perfluoroalkylethyl acrylate expressed by the following formula (III) may be exemplified. 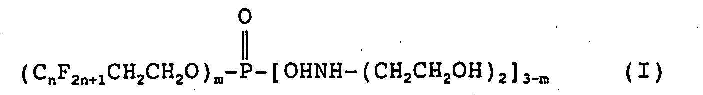 wherein n is an integer of 5 to 20 and m is 1 or 2

CₐF₂ₐ₊₁-(CH₂)_{b}-SiX₃ (II)

wherein a is an integer of 1 to 12, b is an integer of 1 to 5 and X is a halogen atom, an alkyl group or an alkoxy group 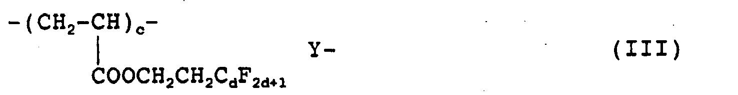 wherein c is an integer of 1 to 12, d is an integer of 5 to 20 and Y is polyethylene glycol or an alkyl copolymer containing a silicone chain, an acryl group, etc.

It is also possible to use a fluorine compound having a perfluoropolyether group such as perfluoropolyether dialkyl phosphoric acid or its salt, perfluoropolyether dialkyl sulfuric acid or its salt, or perfluoropolyether dialkyl carboxylic acid or its salt. Here, the "perfluoropolyether group" means a group having at least two oxygen atoms bonded to perfluoroalkylene or perfluoroalkyl and those having a molecular weight of about 300 to 7000 is preferable from the viewpoints of oil repellency and water repellency.

The method of fluorine treatment is not particularly limited, but, for example, the following method may be mentioned. That is, an aqueous solution of a fluorine compound is gradually added to an aqueous dispersion of the powder to be treated, then an aqueous solution of hydrochloric acid is added thereto to adjust the acidity, the mixture is aged for about 2 hours, and thereafter, the mixture is filtered, dried, and pulverized to obtain the fluorine-treated powder.

Note that, if the surface of the powder is treated to impart fluorine groups and give water repellency and oil repellency, it is also possible to perform other treatment such as to further add other non-fluorine groups, for example, alkyl groups or silicone groups or hydrophilic groups unless the effects of the present invention are not impaired.

In the present invention, before applying the above fluorine treatment, it is also possible to treat the powder surface with at least one gel selected from hydrates, partial dehydrates, and anhydrides of metal hydroxides or metal salts. By performing the fluorine treatment after this metal treatment in this way, it is possible to stably and efficiently promote the formation of a covering of a fluorine compound and produce a fluorine-treated powder superior in water repellency and oil repellency. Further, when using a powder treated with fluorine after metal treatment, the impact stability (impact resistance) is remarkably improved. Further, the effect of improvement of adhesion to the skin etc. is also superior.

The above metal hydroxide or metal salt is preferably a hydroxide or salt of one or more types of metal selected from magnesium, aluminum, silicon, titanium, zinc, zirconium, and barium.

The metal treatment of the powder surface consists, for example, of adding water to the powder to create a slurry, adding, to the resultant slurry, a strongly ionic water soluble compound of the above metal, for example, an aqueous solution of aluminum chloride, sodium aluminate, aluminum sulfate, sodium silicate, magnesium chloride, magnesium sulfate, barium chloride, zirconium chloride, titanium tetrachloride, water-soluble organic titanium, titanyl sulfate, zinc chloride, zinc sulfate, etc., and whereby the powder surface adsorb the compound. Next, an acid or alkali solution is added to cause the compound adsorbed to the powder surface to hydrolyze or be substituted, whereby a hydrate, partial dehydrate, or anhydride of the above metal hydroxide or metal salt is produced.

Next, to the above powder treated on its surface with a metal, an emulsion obtained by adding water to a fluorine compound is gradually added. Then, the emulsion is destroyed with acid or allowing it to stand at a high temperature, whereby the powder covered by the hydrate, partial dehydrate or anhydride of the above metal hydroxide or metal salt can be covered with the fluorine compound.

The less the hydrate, partial dehydrate or anhydride of the above metal hydroxide or metal salt covering the powder the better. The amount is preferably 1 to 30% by weight, as the metal compound producing these, based upon the powder. If the amount is less than 1% by weight, it is difficult to sufficiently bring out the effect of the metal treatment. On the other hand, if the amount is more than 30% by weight, the powder becomes bulky and the inherent functions of the powder tend to be obstructed.

The amount of the fluorine compound used in the fluorine treatment is also preferably 1 to 30% by weight based upon the powder. If the amount is less than 1% by weight, the covering of the fluorine compound is insufficiently formed and it is difficult to obtain the water repellency and oil repellency. On the other hand, if the amount is more than 30% by weight, the powder becomes bulky and the inherent functions of the powder tend to be obstructed.

The powder to be treated is not particularly limited. Various inorganic powders, any powders such as plastic powders of nylon, polyethylene, polypropylene, silicone, etc. can be used. Among them, from the viewpoint of improvement of the feeling of use as a powder cosmetic composition (lack of scratchy feeling, lack of chapping of skin, etc.), a clay mineral powder is preferably used. As the clay mineral powder, any natural or synthetic clay minerals may be used. As the clay mineral powder, talc (hydrous magnesium silicate; 3MgO·₄SiO₂·H₂O), kaolin (hydrous aluminum silicate; Al₂O₃·2SiO₂·2H₂O), silicic anhydride (SiO₂), saponite (hydrous aluminum magnesium silicate; mixture of SiO₂, Al₂O₃, MgO, and water), sericite (microcrystalline hydrous aluminum potassium silicate; K₂O·3Al₂O₃·6SiO₂, 2H₂O), mica (hydrous aluminum potassium silicate, KAl₂·AlSi₃O₁₀(OH)₂), boron nitride (BN), etc. is preferably used. By using these clay minerals, it is possible to blend a larger amount of the later mentioned component (b), while maintaining the powder state and possible to achieve a greater improvement in feeling of use (lack of scratchy feeling, lack of chapping of skin, etc.)

Note that it is also possible to use a composite pigment composed, for example, of a clay mineral coated with titanium oxide etc. such as titanium oxide sericite as the treated powder component.

The form of the powder to be treated is not particularly limited. Any shapes such as a plate shaped, spherical shaped, porous shape may be used. Among these, one of a plate shape is preferably used from the viewpoint of enabling the oil to be efficiently turned into a powder by a small amount of powder.

Further, as the polymer powder where the powder itself contains fluorine groups shown in the above (ii), for example, polytetrafluoroethylene powder etc. may be mentioned.

Note that the powder composition of the present invention may have further blended thereto, in addition to the above component (a), a powder not treated with fluorine or a medicine etc. as a powder as it is to an extent not impairing the effects of the present invention.

The component (b) is composed of one or more types of oily components. The surface tension value (average) is at least 2.0 x 10⁻² N/m (with respect to the atmosphere), preferably at least 2.1 x 10⁻² N/m. If the surface tension value (average) of the component (b) is less than 2.0 x 10⁻² N/m, the component (b) will easily wet the powder, so the component (b) will not be able to be converted to a good powder form.

Note that here, the "surface tension value (average) of the component (b)" means the surface tension value of the component (b) as a whole which forms the liquid. So long as the surface tension value of the component (b) as a whole satisfies the above range, it is possible to use as ingredients of the component (b) oily components having a surface tension value of less than 2.0 x 10⁻² N/m. Therefore, the component (b) can contain oily components of any properties such as solid, semisolid, and liquid ingredients. Further, the component (b) includes not only homogeneously dissolving ingredients, but also a dispersion composed of an oil dispersed in an oil.

As the component (b), preferably, for example, hydrocarbon oils such as liquid paraffin, ozokerite, squalene, pristane, paraffin, ceresin, squalane, and vaseline; polar oils such as liquid lanolin, isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, 12-cholesterol hydroxystearate, ethylene glycol di-2-ethylhexanoate, dipentaerythrite fatty acid ester, N-alkylene glycol monoisostearate, neopentyl glycol dicaprate, diisostearyl malate, glyceryl di-2-heptyl undecanoate, trimethylolpropane tri-2-ethylhexanoate, trimethylolpropane triisostearate, pentaerythritol tetra-2-ethylhexanoate, glyceryl tri-2-ethylhexanoate, trimethylolpropane triisostearate, cetyl-2-ethylhexanoate, 2-ethylhexyl palmitate, glyceryl trimyristate, glyceryl tri-2-heptyl undecanoate, castor oil fatty acid methyl ester, oleic acid, acetoglyceride, 2-heptylundecyl palmitate, diisopropyl adipate, 2-octyldodecyl-N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, triethyl citrate; higher alcohols such as stearyl alcohol, behenyl alcohol, oleyl alcohol, cetostearyl alcohol; plant oils such as alcohols, jojoba oil, olive oil, nut oil, safflower oil, soybean oil, etc. may be mentioned. Among these, particularly preferred are liquid paraffin or plant oils such as jojoba oil having relatively large surface tension value.

Small surface tension value Silicone oils such as, for example, chain type silicones such as dimethyl polysiloxane, methylphenyl polysiloxane, methylhydrogenpolysiloxane; ring type silicones such as octamethylcyclotetrasiloxane, decamethylcyclopentasiloxane, dodecamethyl-cyclohexasiloxane, fluorocarbon oils as represented by the "Fomblin" series (made by Ausimont), and fragrances such as limonene having can also be used in small amounts in a range of the surface tension value of the component (b), as a whole, of not less than 2.0 x 10⁻² N/m. When formulating a large amount of silicone oil or fluorocarbon oil having a small surface tension value, the surface tension value will sometimes become too small and the oily component will not be able to be sufficiently converted to a powder. The surface tension value can be determined by the known method such as Pendant Drop Method.

Further, semisolid oily components such as hydrogenated palm oil, hydrogenated castor oil, vaseline, solid oils such as paraffin wax, microcrystalline wax, carnauba wax, candelilla wax, also can be used to an extent where the surface tension value of the ingredient (b) as a whole would satisfy the above range.

In particular, in the case of a powder cosmetic composition, the ingredient (b) is preferably an oily component having a melting point of about 37°C or less which would therefore dissolve on the skin, but small amounts of solid oils such as synthetic waxes, natural waxes can also be blended.

Note that it is also possible to formulate, into the oily component, oil soluble vitamin such as vitamin A and its derivatives, sterols, natural and synthetic fragrances, UV absorbers, substances difficult to be dissolved in water, etc. to an extent not impairing the effect of the present invention.

In the present invention, the formulation amount of the component (a) is preferably 10 to 80% by weight, more preferably 20 to 70% by weight, based upon the total amount of the composition. If the formulated amount is too small, it is not possible to sufficiently convert the oily component of the component (b) into a powder and, therefore, may no longer be possible to obtain the desired powder form. On the other hand, if the formulated amount is too large, liquification will become difficult even with the friction of an application thereof during the use and the powder will become organoleptically unpreferable.

Further, when including a powder component other than the component (a), the formulation ratio of the component (a) to the entire powder components is preferably 50 to 100% by weight.

The formulated amount of the component (b) may be made the residual amount of the total amount of the other necessary components and optional additive components included in the powder composition of the present invention, but in the present invention, 20 to 90% by weight is preferable. If the amount of the component (b) is too small, liquification will be difficult by the friction of an application thereof and the powder will become organoleptically undesirable. On the other hand, if the formulation amount is too large, the formation of the powder will becomes difficult. In the present invention, in particular when using a clay mineral as the powder component (a), it is possible to formulate a higher amount of the component (b) while maintaining the powder state. In particular, in the case of a powder cosmetic composition, it is possible to obtain a superior feeling of use suppressing the scratchy feeling, sticky feeling, and chapping feeling.

In the present invention, the ratio (weight ratio) of the component (a) to the component (b) is preferably 1:9 to 4:1, more preferably 1:5 to 3:1.

The powder composition of the present invention, particularly in the case of a powder cosmetic composition, may further have formulate therein, in addition to the above components, various types of any ingredients usable in ordinary cosmetics such as fragrances, various types of powders, oil soluble medicinal ingredients, etc. to an extent not impairing the effects of the present invention.

The powder composition of the present invention does not substantially contain any water. In particular, when used as a powder cosmetic composition, it may be prepared, without formulating additive ingredient such as any preservative. A cosmetic composition meeting with the recent consumer desire for safety in the cosmetic industry therefore becomes possible.

Further, the powder composition of the present invention may be prepared, without formulating in any surfactant. However, it is also possible to suitably formulate a surfactant in accordance with the application of the cosmetic composition such as a powder type partial makeup remover. A powder type makeup remover containing a surfactant as an additive ingredient would have a high makeup removal effect, would be easy to use with no dripping, and could be washed off by water. When using a surfactant for a powder type partial makeup remover, the formulation amount of the surfactant is preferably about 0.1 to 40% by weight based upon the component (b). As the surfactant, any of a nonionic surfactant, an anionic surfactant and a cationic surfactant can be formulated. As the type of the surfactant, it may be either hydrophilic or lyophilic. It is also possible to disperse and formulate in the oil phase in the form of an emulsion or a solid powder a surfactant having the property of not dissolving in an oil phase.

The powder composition of the present invention is composed of the component (b) adsorbing the component (a) around it and having the component (a) cause the component (b) to turn into powder. By applying force by the friction of an application thereof, this adsorbed state is destroyed and the powderized component (b) seeps out and liquifies. Further, the feeling of use and effectiveness of the component (b) are manifested.

The powder composition of the present invention is not particularly limited in the production method thereof so long as the component (b) adsorbs the component (a) around it and the component (a) causes the component (b) to become a powder. For example, the production method of adding and mixing the component (a) to the component (b) or this component with any additional ingredients dissolved therein was mentioned, but the invention is not limited thereto.

The powder cosmetic composition of the present invention containing the above components (a) and (b) as essential ingredients can reduce the scratchy feeling which had been considered an inconvenience specific to powder cosmetics in the past and gives superior effects in terms of feeling of use. Further, the powderization is good, the powder easily liquifies at the time of the friction of an application thereof, and a fresh feeling of use is given.

In a third aspect of the present invention, an unstable ingredient easily creating an odor, spoiling, etc. in the present of light, oxygen, water, etc. is formulate as the component (c) in addition to the above components (a) and (b).

A component unstable in the presence of light, oxygen, or water of the component (c) means, for example, an ingredient which is blended as a medicine etc. in a cosmetic, pharmaceutical, etc., is unstable in contact with water or with respect to light, heat, oxygen, etc., and breaks down, loses activity, discolors or fades, or produces odor etc. when formulated as it is in a cosmetic.

As such an ingredient, water soluble medicinal ingredients or oil soluble medicinal ingredients may be mentioned as typical examples. Specifically, a whitener, anti-inflammatory agent, humectant, blood circulation promoter, hair tonic, antibacterial agent, hormone, vitamin, enzyme, antioxidant, animal or plant extract, or other medicine may be mentioned.

As a whitener, hydroxyquinone derivative such as albutin, kojic acid, L-ascorbic acid and its derivatives, γ-oryzanol, ellagic acid, lucinol, etc. may be mentioned.

L-ascorbic acid is generally called vitamin C. Due to its strong reducing action, it has a cell respiratory action, enzyme activating action, and collagen forming action and has a melanin reducing action. As an L-ascorbic acid derivative, for example, L-ascorbic acid monoesters such as L-ascorbic acid monophosphoric acid ester, L-ascorbic acid-2-sulfuric acid ester, L-ascorbic acid glucosides such as L-ascorbic acid-2-glucoside; or salts thereof (L-ascorbic acid magnesium phosphate etc.) etc. may be mentioned.

As an anti-inflammatory agent, for example, a glycyrrhizinic acid salt (for example, dipotassium glycyrrhizinate, ammonium glycyrrhizinate, etc.), allatoin, hinokitiol, tranexemic acid and its derivatives, etc. may be mentioned. As a tranexemic acid derivative, a dimer of tranexemic acid (for example, hydrochlorate trans-4-(tranaminomethylcyclohexanecarbonyl)aminomethylcyclohexacarboxylic acid etc.), an ester of tranexemic acid and hydroquinone (for example, trans-4-aminomethylcyclohexanecarboxylic acid 4'-hydroxyphenylester etc.), an ester of tranexemic acid and gentisic acid (for example, 2-(trans-4-aminomethylcyclohexylcarbonyloxy)-5-hydroxybenzoic acid and its salts etc.), an amide of tranexemic acid (for example, methylamide trans-4-aminomethylcyclohexanecarboxylate and its salts, trans-4-(P-methoxybenzoyl)aminomethyl-cyclohexanecarboxylic acid and its salts, trans-4-guanidinomethylcyclohexanecarboxylic acid and its salts, etc.), etc. may be mentioned.

As a humectant, for example, ceramide and its derivatives, amino acid salts, urea, linolic acid, linoleic acid, trimethylglycine, etc. may be mentioned.

As a blood circulation promoter and hair tonic, for example, capcaisin, caffeine, acetyl choline, tannic acid, minoxidil, etc. may be mentioned.

As an antibacterial agent, for example resorcin, sulfur, salicylic acid, benzalkonium chloride, etc. may be mentioned.

As a hormone, for example, oxytocin, corticotropin, vasopressin, secretin, gastrin, calcitonin, estradiol and its esters, cortisone and its esters, etc. may be mentioned.

As a vitamin, for example, vitamin A and its derivatives, vitamin B₆, vitamin B₆ hydrochlorate, and other vitamin B₆ derivatives, vitamin B₁₂ and its derivatives, nicotinic acid, nicotinamide, and other nicotinic acid derivatives, vitamin D and its derivatives, vitamin E and its derivatives, vitamin H and its derivatives, pantothenyl ethyl ether, etc. may be mentioned.

As an enzyme, for example, trypsin, lysozyme chloride, chemotrypsin, semialkali protease, serapeptase, lipase, hyaluronidase, etc. may be mentioned.

As an antioxidant, thiotaurin, glutathion, catechin, albumin, ferritin, metallothionein, etc. may be mentioned.

As an animal and plant extract, tea extract, *rosa roxburghii plena* extract, *scutellaria* root extract, saururaceae extract, phellodendron bark extract, sweet clover extract, hypericum extract, licorice extract, peony root extract, soap wort extract, sponge gourd extract, cinchona extract, saxifrage extract, sophora root extract, nuphar extract, fennel extract, *primula sieboldii* extract, rose extract, rehmannia root extract; lemon extract, lithospermum extract, aloe extract, calamus rhizome extract, eucalyptus extract, horse tail extract, sage extract, thyme extract, seaweed extract, cucumber extract, clove extract, raspberry extract, melissa extract, ginseng extract, horse chestnut extract, peach extract, peach leaf extract, mulberry bark extract, cornflower extract, witch hazel extract, licorice extract, ginkgo extract, ichiyaku extract, and other plant extracts and placenta extract, collagen, and other animal extracts etc. may be mentioned.

In the present invention, since the component (a) covers the component (b) (drops of oil), when the component (c) is an oil-soluble medicine, it is possible to block out light more effectively than with just the state of the oil drops.

Further, the present invention provides a system substantially not containing any water, so when the component (c) is a water soluble medicine, it is formulated in the form of a powder rather than an aqueous solution. Therefore, the component (c) is not affected by the radicals and the stability can in particular be strikingly improved.

Note that, due to the component (a) covering the component (b), the permeation of oxygen to the component (b) will be somewhat suppressed, but the covering of then component (a) seen microscopically cannot completely block out oxygen, so the extent of the effect of stabilization is somewhat lower for the stabilization of medicine unstable with respect to oxygen than stabilization of medicine unstable with respect to light and water. In practice, however, it is of an extent not posing any problem, and the desired effect of the present invention can be fully brought out.

When the component (c) is an oil soluble ingredient, it is blended by dissolving it in the component (b). Further, when the component (c) is a water soluble ingredient, it is formulated by dispersing it in the component (b) or dispersing it in the component (a). A medicine is extracted by a solvent (ethanol etc.) and formulated the oil or formulated by reducing the plant to a powder.

The formulation amount of the component (c) need only be one of an extent enabling the effect of the active ingredient contained in it to be fully manifested and is not particularly limited, but about 0.001 to 10% by weight based upon the entire weight of the powder cosmetic composition is preferable, about 0.005 to 7% by weight is more preferable. If the formulation amount is too small, the function of the component (c) serving as the active ingredient is hard to fully manifest, while if the formulation amount is larger than necessary, it would be difficult to expect any increase in effect commensurate with the increase in the amount. With the powder cosmetic composition of the present invention, even if containing an unstable component (c), the component can be stabilized and its functions fully manifested.

The powder cosmetic composition of the present invention may contain, in addition to the above components, various types of any ingredients usable in ordinary cosmetics, for example, humectants, surfactants, fragrances, various types of powders and also various types of solvents such as ethanol to an extent not impairing the effects of the present invention.

The cosmetic composition of the present invention is a powder form cosmetic composition, but liquifies upon the friction of an application thereof. It has no sticky feeling, can suppress chapping of the skin, can effectively prevent adhesion of the oil or dirt etc. on the container in spite of an oil-rich preparation, and can stably hold even unstable ingredients which easily break down, spoil, etc. in the present of light, oxygen, water, etc.

The powder cosmetic composition of the present invention is composed of the component (b) and further the component (c) adsorbing the component (a) around them and having the component (a) cause the component (b) and the component (c) turn into powder. By applying force by the friction of an application thereof, this adsorbed state is destroyed and the powderized components (b) and (c) seep out and liquify. Further, the feeling of use of the component (b) is improved and the effectiveness of the component (c) is manifested.

The powder cosmetic composition of the present invention is not particularly limited in the method of its production so long as the component (b) and further the component (c) adsorb the component (a) around them and the component (a) causes the component (b) and the component (c) to become a powder. For example, the method of production of adding and mixing the component (a) to the component (b) or this component with the component (c) dissolved in it was mentioned, but the invention is not limited thereto.

Note that, when the composition of the present invention is applied to a powder paint, there is the advantage that it is possible to use, for example, an oil dye as the oily component and cover this homogeneously by fluorine-treated powder so as to enable the paint of the powder form to be easily applied to a wall, paper, etc. by a brush etc. by just the friction of an application thereof.

### EXAMPLES

The present invention will now be explained in further detail based on Examples, but of course the present invention is not limited to the following Examples. Note that the amounts blended are shown by % by weight unless otherwise particularly noted.

Before the Examples, the test methods and evaluation methods used in the present invention will be explained.

### Feeling of Use (Lack of scratchy Feeling

The feelings of use (lack of scratchy feeling) of the products of the Examples and the products of the Comparative Examples were judged and evaluated by actual usage tests by a panel (50 members) using the following criteria:

### Criteria for Judgment

Remarkably effective: Not scratchy
Effective: Slightly scratchy, but of extent not problem in use
Somewhat effective: Somewhat scratchy
Ineffective: Remarkably scratchy

### Evaluation

++: At least 80% of test subjects evaluated product as remarkably effective, effective, or somewhat effective
+: At least 50%, but less than 80% of test subjects evaluated product as remarkably effective, effective, or somewhat effective
±: At least 30%, but less than 50% of test subjects evaluated product as remarkably effective, effective, or somewhat effective
-: Less than 30% of test subjects evaluated product as remarkably effective, effective, or somewhat effective

### Feeling of Use (Lack of Sticky Feeling)

The feelings of use (lack of sticky feeling) of the products of the Examples and the products of the Comparative Examples were judged and evaluated by actual usage tests by a panel (50 members) using the following criteria:

### Criteria for Judgment

Remarkably effective: Not sticky
Effective: Slightly sticky, but of extent not problem in use
Somewhat effective: Somewhat sticky
Ineffective: Remarkably sticky

### Evaluation

++: At least 80% of test subjects evaluated product as remarkably effective, effective, or somewhat effective
+: At least 50%, but less than 80% of test subjects evaluated product as remarkably effective, effective, or somewhat effective
±: At least 30%, but less than 50% of test subjects evaluated product as remarkably effective, effective, or somewhat effective
-: Less than 30% of test subjects evaluated product as remarkably effective, effective, or somewhat effective

### Feeling of Use (Freshness)

The feelings of use (freshness) of the products of the Examples and the products of the Comparative Examples were judged and evaluated by actual usage tests by a panel (50 members) using the following criteria:

### Criteria for Judgment

Remarkably effective: Extremely fresh
Effective: Fresh
Somewhat effective: Somewhat fresh
Ineffective: Not fresh

### Evaluation

++: At least 80% of test subjects evaluated product as remarkably effective, effective, or somewhat effective
+: At least 50%, but less than 80% of test subjects evaluated product as remarkably effective, effective, or somewhat effective
±: At least 30%, but less than 50% of test subjects evaluated product as remarkably effective, effective, or somewhat effective
-: Less than 30% of test subjects evaluated product as remarkably effective, effective, or somewhat effective

### Effect of Suppression of Chapping

The effects of suppression of chapping of the products of the Examples and the products of the Comparative Examples were judged and evaluated by actual usage tests by a panel (50 members) using the following criteria:

### Criteria for Judgement

Remarkably effective: Chapping extremely suppressed Effective: Chapping somewhat suppressed
Somewhat effective: Chapping somewhat suppressed, but not that much change
Ineffective: Chapping became worse

### Evaluation

++: At least 80% of test subjects evaluated product as remarkably effective, effective, or somewhat effective
+: At least 50%, but less than 80% of test subjects evaluated product as remarkably effective, effective, or somewhat effective
±: At least 30%, but less than 50% of test subjects evaluated product as remarkably effective, effective, or somewhat effective
-: Less than 30% of test subjects evaluated product as remarkably effective, effective, or somewhat effective

### Stability Test of Medicines (Ingredients Unstable in Presence of Light, Oxygen, Water, Etc.)

The remaining amounts of the unstable ingredients when stored at 50°C for 2 weeks were measured by HPLC for the products of the Examples and the products of the Comparative Examples and the residual rates (% by weight) were investigated from there.

### Effect of Suppression of Adhesion of Dirt to Container

The degrees of adhesion of dirt to the containers with use for one month were judged and evaluated for the products of the Examples and the products of the Comparative Examples by actual usage tests by a panel (50 members) using the following criteria:

### Criteria for Judgment

Remarkably effective: Oil or other dirt does not adhere to container
Effective: Some dirt adheres, but of extent not problem in use
Somewhat effective: Somewhat dirty
Ineffective: Remarkably dirty

### Evaluation

++: At least 80% of test subjects evaluated product as remarkably effective, effective, or somewhat effective +: At least 50%, but less than 80% of test subjects evaluated product as remarkably effective, effective, or somewhat effective
±: At least 30%, but less than 50% of test subjects evaluated product as remarkably effective, effective, or somewhat effective
Less than 30% of test subjects evaluated product as remarkably effective, effective, or somewhat effective

### Surface Tension Value of Oily Component

### Measured by Pendant Drop Method.

### Impact Stability (Impact Resistance)

A 20 ml glass sample tube was filled with a product of each Example up to its top. This was dropped 10 times on a rubber sheet from a height of 70 cm, then the inside state was observed visually and evaluated by the following criteria:

### Evaluation

++: No change (powder form maintained)
+: Some paste-like substance adhered to tube, but no problem overall
±: Noticable adhesion of paste-like substance
-: Entire amount became paste-like

### Example I-1 and Comparative Examples I-1 and I-2

Powder cosmetics were prepared by the compositions shown in the following Table I-1. The above test methods were used to evaluate the feeling of use (lack of scratchy feeling and lack of sticky feeling), the effect of suppression of chapping, and the effect of suppression of adhesion of dirt to the container of Example I-1 and Comparative Examples I-1 and I-2. Note that in Table I-1, the fluorine-treated talc (*1) used is "PF-5 JA46R" (made by Daito Kasei). Further, the perfluoropolymethylisopropylether (**) used is "Fomblin HC/O4" (made by Ausimont). The results are shown in Table I-1.

**Table I-1**

| Formulation ingredient | Ex. I-1 | Comp. Ex. I-1 | Comp. Ex. I-2 |
|---|---|---|---|
| (1) Fluorine-treated talc (*) | 48.9 | - | 94.5 |
| (2) Squalane | 50 | 50 | 5 |
| (3) Perfluoropolymethyl-isopropylether (**) | 0.5 | 0.5 | 0.5 |
| (4) Lavender oil | 0.05 | 0.05 | - |
| (5) Vitamin E acetate | 0.05 | 0.05 | - |
| (6) Dimethyl silicone | 0.5 | 49.4 | - |
| Feeling of use (lack of scratchy feeling) | ++ | ++ | - |
| Feeling of use (lack of sticky feeling) | ++ | ± | ++ |
| Effect of suppression of chapping | ++ | ++ | - |
| Effect of suppression of adhesion of dirt to container | ++ | - | ++ |

### Production Method

The ingredients (3) to (9) were mixed and dissolved. The oil phase was then added to the powder of the ingredient (1), then the result was mixed, stirred, and filled in a container. Note that the surface tension value of the (b) ingredient of Example I-1 was 2.7 x 10⁻² N/m.

As clear from the results of Table I-1, the powder cosmetic of Example I-1 had the effect of suppression of chapping and exhibited an extremely good feeling of use with no scratchy feeling or sticky feeling. Also, no dirt was observed on the container.

### Example I-2 and Comparative Example I-3

The compositions shown in the following Table I-2 were used to visually evaluate the ability to form a powder. Note that in Table I-2, the fluorine-treated sericite (*) used was "NFP Sericite" (made by Daikin Industries) comprised of sericite treated on its surface by a perfluoroalkylphosphate ester, a butylacrylate/perfluoroalkylethylacrylate/ mercaptopropyldimeticon copolymer, and a perfluoroalkylethylacrylate/HEMA copolymer. Further the dimethyl silicone (**) used was "Silicone KF96A-6" (made by Toray Silicone). The results are shown in Table I-2.

**Table I-2**

| Formulation ingredient | Ex. I-2 | Comp. Ex. I-3 |
|---|---|---|
| (1) Fluorine-treated sericite (*) | 35 | 35 |
| (2) Dimethyl silicone (**) | - | 64.8 |
| (3) Jojoba oil | 64.8 | - |
| (4) Vitamin A acetate | 0.05 | 0.05 |
| (5) Dibutyl hydroxytoluene | 0.05 | 0.05 |
| (6) Butyl parabene | 0.1 | 0.1 |
| Surface tension value of oily component (N/m) | 3.2 x 10⁻² | 1.9 x 10⁻² |
| Powderization | + | ± (powder state not achieved) |

### Production Method

The ingredients (2) to (6) were mixed and dissolved, then the result was added to the ingredient (1). The result was then mixed, stirred, then filled in a container.

As clear from the results of Table I-2, in Comparative Example I-3, the surface tension value of the oil was low and a powder could not be formed.

### Example I-3 and Comparative Example I-4

The compositions shown in the following Table I-3 were used to visually evaluate whether a powder could be formed. Note that in Table I-3, the fluorine-treated titanium oxide (*) used was "FSA-52 Titan CR-50) (made by Daito Kasei) comprised of titanium oxide treated on its surface by perfluoroalkylphosphate ester and an acrylate/dimeticon copolymer. Further, the dimethyl silicone oil treated anhydrous silicate (**) used was "Aerosil R202" (made by Aerosil Japan, surface volume 100 m²/g). The results are shown in Table I-3.

**Table I-3**

| Formulation ingredient | Ex. I-3 | Comp. Ex. I-4 |
|---|---|---|
| (1) Fluorine-treated titanium oxide (*) | 40 | - |
| (2) Dimethyl silicone oil treated anhydrous silicate (**) | - | 40 |
| (3) Macademia nut oil | 35 | 35 |
| (4) Octylmethoxycinnamate | 20 | 20 |
| (5) 1,3-butylene glycol | 5 | 5 |
| Powderization | + | - (powder state not achieved) |

### Production Method

The ingredients (3) to (5) were homogeneously mixed. Next, the result was mixed and stirred with the ingredient (1) or ingredient (2) and the result filled in a container.

As clear from the results of Table I-3, a powder not treated with fluorine such as in Comparative Example I-4 could not achieve powderization of the oil. Further, it is possible to add a humectant or other ingredient dissolving in the oil to an extent not overly affecting the surface tension of the oil. Note that the surface tension value of the component (b) of Example I-3 was 3.3 x 10⁻² N/m.

### Examples I-4 and I-5

Powder cosmetics were prepared by the compositions shown in the following Table I-4. The above test methods were used to evaluate the feeling of use (lack of scratchy feeling), feeling of use (lack of sticky feeling), effect of suppression of chapping, and effect of suppression of adhesion of dirt on the container of Examples I-4 and I-5. Note that in Table I-4, the fluorine-treated talc (*) used was "Novatec NFP Talc" (made by Daikin Industries), while the fluorine-treated mica (**) used was "PF-5 Mica MJV") (made by Daito Kasei). The results are shown in Table I-4.

**Table I-4**

| Formulation ingredient | Ex. I-4 | Ex. I-5 |
|---|---|---|
| (1) Fluorine-treated talc (*) | 46.8 | 36.8 |
| (2) Fluorine-treated mica (**) | - | 10 |
| (3) Vaseline | 3 | 3 |
| (4) Macademia nut oil | 50 | 50 |
| (5) Dibutyl silicone | 0.2 | 0.2 |
| Surface tension value of oily component (N/m) | 2.7 x 10⁻² | 2.7 x 10⁻² |
| Evaluation of feeling of use (lack of scratchy feeling) | + | ++ |
| Evaluation of feeling of use (lack of sticky feeling) | ++ | ++ |
| Effect of suppression of chapping | ++ | ++ |
| Effect of suppression of adhesion of dirt to container | ++ | ++ |

### Production Method

The ingredients (3) to (5) were mixed and dissolved, then the result was added to the ingredients (1) and (2), then mixed and stirred and the result filled into a container.

As clear from the results of Table I-4, in a powder cosmetic using a fluorine-treated clay mineral powder, there is no scratchy feeling or sticky feeling, the effect of suppression of chapping is excellent, and the effect of suppression of adhesion of dirt to the container is excellent. Further, it is possible to blend in a large amount of oily component.

### Example I-6

A powder composition was prepared by the composition shown in the following Table I-5. The above test methods were used to evaluate the feeling of use (lack of sticky feeling) and the effect of suppression of adhesion of dirt to the container of Example I-6. Note that in Table I-5, the fluorine-treated microparticulate titanium oxide (*) used was "Novatec NFP TiO₂" (made by Daikin Industries). The results are shown in Table I-5.

**Table I-5**

| Formulation ingredient | Ex. I-6 |
|---|---|
| (1) Fluorine-treated microparticulate titanium oxide | 77 |
| (*) | 3 |
| (2) Vaseline | 20 |
| (3) Macademia nut oil | |
| Surface tension value (N/m) of oily component | 3.0 x 10⁻² |
| Evaluation of feeling of use (lack of sticky feeling) | ++ |
| Effect of suppression of adhesion of dirt to container | ++ |

### Production Method

The ingredients (2) to (3) were mixed and dissolved, then the result added to the ingredient (1). This was then mixed, stirred, and filled in a container.

As shown in Table I-5, when using a non-clay mineral as the treated powder ingredient, it was possible to obtain a lack of sticky feeling and effect of suppression of adhesion of dirt to the container, but the amount of the oily component blended could not be made as high as with use of a clay mineral as the treated powder ingredient.

### Example I-7: Moisture Retaining Powder

| Formulation ingredient | % by weight |
|---|---|
| Fluorine-treated sericite ("PF-5 | 36.5 |
| Eightpearl 300S-Al", made by Daito Kasei) Beeswax | 0.5 |
| Vaseline | 1 |
| Squalane | 40 |
| Pentaerythrityl tetraoctanoate | 22 |

### Evaluation

A moisture retaining powder of the above composition was prepared. The surface tension value of the oily component of the powder was 2.6 x 10⁻² N/m. The powder was used applying the above test methods to evaluate the feeling of use (lack of scratchy feeling), feeling of use (lack of sticky feeling), effect of suppression of chapping, and effect of suppression of adhesion of dirt to the container, whereupon the following was found:
Feeling of use (lack of scratchy feeling): ++
Feeling of use (lack of sticky feeling): ++
Effect of suppression of chapping: ++
Effect of suppression of adhesion of dirt to the container: ++

### Example I-8: Foundation

| Formulation ingredient | % by weight |
|---|---|
| Fluorine-treated sericite ("PF-5 Eightpearl 300S-Al" made by Daito Kasei) | 37 |
| Petal shaped zinc oxide | 3 |
| Liquid paraffin | 59 |
| Octylmethoxycinnamate | 1 |

### Evaluation

A foundation of the above composition was prepared.

The surface tension value of the oily component of the foundation was 3.0 x 10⁻² N/m. The foundation was used applying the above test methods to evaluate the feeling of use (lack of scratchy feeling), feeling of use (lack of sticky feeling), effect of suppression of chapping, and effect of suppression of adhesion of dirt to the container, whereupon the following was found:
Feeling of use (lack of scratchy feeling): ++
Feeling of use (lack of sticky feeling): ++
Effect of suppression of chapping: ++
Effect of suppression of adhesion of dirt to the container: ++

### Example I-9

The fluorine-treated powder and metal-treated and fluorine-treated powder shown in the following Table I-6 were used to measure oil repellency and maximum amount of oil blended by the following methods:

### Oil Repellency

The powder was converted into pellets and the contact angle when liquid paraffin was dropped from above was measured. The larger the contact angle, the better the oil repellency.

### Maximum Formulation Amount of Oily Component

The maximum amount of oily component blended able to maintain the powder form (here, liquid paraffin used) was measured for 1 g of powder.

Note that in Table I-6, the fluorine-treated talc (*1) used was "PF-5 Talc JA-46R", the alumina-treated fluorine-treated talc (*2) used was "PF-5 Talc JA-46R-AL", the fluorine-treated sericite (*3) used was "PF-5 Sericite FSA", and the alumina-treated fluorine-treated sericite (*4) used was "PF-5 Eightpearl 300S-AL". Further, the zinc-treated fluorine-treated sericite (*5), magnesium-treated fluorine-treated sericite (*6), barium fluorine-treated sericite (*7), and zirconium-treated fluorine-treated sericite (*8) used were powders obtained by using ZnSO₄, MgCl₂·6H₂O, BaCl₂·6H₂O, and ZrOCl₂·8H₂O to treat the surface of sericite, then treated by fluorine. The results are shown in Table I-6.

**Table I-6**

| Formulation ingredient | Oil repellency (contact angle) | Maximum amount of oily component blended (with respect to 1 g of powder) |
|---|---|---|
| Fluorine-treated talc (*1) | 108° | 1.6 g |
| Alumina-treated fluorine-treated talc (*2) | 143° | 2.4 g |
| Fluorine-treated sericite (*3) | 119° | 2.1 g |
| Alumina-treated fluorine-treated sericite (*4) | 150° | 3.6 g |
| Zinc-treated fluorine-treated sericite (*5) | 138° | 3.0 g |
| Magnesium-treated fluorine-treated sericite (*6) | 148° | 3.4 g |
| Barium fluorine-treated sericite (*7) | 143° | 3.2 g |
| Zirconium-treated fluorine-treated sericite (*8) | 151° | 3.6 g |

As clear from the results of Table I-6, by applying metal treatment, the oil repellency is remarkably improved. Further, it is learned that the amount of oil which can be encapsulated in the powder largely increases.

### Examples I-10 to I-13

Powder compositions were prepared by the compositions shown in the following Table I-7. The above test methods were used to evaluate the feeling of use (lack of scratchy feeling and lack of sticky feeling), the effect of suppression of chapping, and the impact resistance of Examples I-10 to I-13. Note that in Table I-7, the fluorine-treated sericite (*) used was "PF-5 Sericite FSA" (made by Daito Kasei), while the alumina-treated fluorine-treated sericite (**) used was "PF-5 Eightpearl 300S-AL". The results are shown in Table I-7.

**Table I-7**

| Formulation ingredient | Ex. I-10 | Ex. I-11 | Ex. I-12 | Ex. I-13 |
|---|---|---|---|---|
| (1) Fluorine-treated sericite (*) | 50 | - | 30 | - |
| (2) Alumina-treated fluorine-treated sericite (**) | - | 50 | - | 30 |
| (3) Jojoba oil | 49 | 49 | 69 | 69 |
| (4) Glycyrrhiza flavonoid | 1 | 1 | 1 | 1 |
| Feeling of use (lack of scratchy feeling) | + | + | ++ | ++ |
| Feeling of use (lack of sticky feeling) | ++ | ++ | + | + |
| Effect of suppression of chapping | + | + | ++ | ++ |
| Impact stability (impact resistance) | ++ | ++ | + | ++ |

### Production Method

The ingredients (3) to (4) were mixed and dissolved, then the result added to the ingredient (1) or (2). The mixture was then mixed and stirred and filled into a container.

As will be clear from Table I-7, the powder composition of the present invention has superior feeling of use (lack of scratchy feeling and lack of sticky feeling), effect of suppression of chapping, and effect of impact stability (impact resistance). In particular, if a fluorine-treated powder which has been treated by a metal is used, an extremely superior impact stability can be obtained even if including some encapsulated oil.

### Example I-14: Eyeshadow

| Formulation ingredients | % by weight |
|---|---|
| Alumina-treated fluorine-treated sericite ("PF-5 Eightpearl 300S-Al", made by Daito Kasei) | 10 |
| Alumina-treated fluorine-treated mica | 30 |
| Fluorine-treated talc | 5 |
| Fluorine-treated polyethylene | 3 |
| Fluorine-treated titanium oxide | 3 |
| Yellow iron oxide | 0.8 |
| Bengara | 0.5 |
| Red iron oxide | 0.7 |
| Pearl agent | 8 |
| Liquid paraffin | 38 |
| Octylmethoxycinnamate | 1 |

### Evaluation

A foundation of the above composition was prepared. The surface tension value of the oily component of this foundation was 3.0 x 10⁻² N/m. The foundation was used applying the above test methods to evaluate the feeling of use (lack of scratchy feeling), feeling of use (lack of sticky feeling), effect of suppression of chapping, effect of suppression of adhesion of dirt to the container, and impact stability (impact resistance), wherein the following results were obtained:
Feeling of use (lack of scratchy feeling): +
Feeling of use (lack of sticky feeling): ++
Effect of suppression of chapping: +
Effect of suppression of adhesion of dirt to the container: ++
Impact stability (impact resistance): ++

Further, the adhesion power to the skin was also superior.

### Example I-15: Powder Perfume

| Formulation ingredients | % by weight |
|---|---|
| Alumina-treated fluorine-treated mica | 30 |
| Alumina-treated fluorine-treated sericite | 15 |
| Fluorine-treated PMMA powder | 4 |
| Hydrophobic starch | 1 |
| Squalane | 45 |
| Fragrance | 5 |

### Evaluation

The powder perfume of the above composition was prepared. The surface tension value of the oily component of the powder perfume was 2.5 x 10⁻² N/m. The powder perfume was not sticky despite being alcohol free and could be picked up by the fingers and applied in a pinpoint manner.

### Example I-16: Powder Sunscreen

| Formulation ingredients | % by weight |
|---|---|
| Alumina-treated fluorine-treated titanium oxide | 10 |
| Alumina-treated fluorine-treated mica | 10 |
| Alumina-treated fluorine-treated sericite | 20 |
| Fluorine-treated zinc oxide | 10 |
| Fluorine-treated silicone resin | 3 |
| Cetyl octanoate | 17 |
| Octylmethoxysuccinate | 30 |

A powder sunscreen of the above composition was prepared. The surface tension value of the oily component of this powder sunscreen was 2.5 x 10⁻² N/m. The powder sunscreen was used applying the above test methods to evaluate the feeling of use (lack of scratchy feeling), feeling of use (lack of sticky feeling), effect of suppression of chapping, effect of suppression of adhesion of dirt to the container, and impact stability (impact resistance), wherein the following results were obtained:
Feeling of use (lack of scratchy feeling): ++
Feeling of use (lack of sticky feeling): ++
Effect of suppression of chapping: ++
Effect of suppression of adhesion of dirt to the container: ++
Impact stability (impact resistance): ++

Further, the adhesion power to the skin was also superior.

### Example I-17: Powder Partial Makeup Remover

| Formulation ingredients | % by weight |
|---|---|
| Alumina-treated fluorine-treated sericite | 40 |
| Pentaerythrityl tetraoctanoate | 25 |
| Cetyl octanoate | 20 |
| Methylphenyl polysiloxane | 5 |
| Isononyl isononate | 2 |
| Polyethyleneglycol diisostearate | 2 |
| Polyethyleneglycol monostearate | 1 |

A powder partial makeup remover of the above composition was prepared. The surface tension value of the oily component of the powder partial makeup remover was 2.3 x 10⁻² N/m. The powder partial makeup remover included an oil cleansing agent in the powder, so had a high makeup removing effect and could be picked up by the hand and applied in a pinpoint manner without dripping like in conventional oil cleansing. Further, since a surfactant is included, the remover could be washed off by water.

### Example I-18: Powder Paint

| Formulation ingredients | % by weight |
|---|---|
| Fluorine-treated sericite | 25 |
| Iron oxide (red) | 25 |
| Liquid paraffin | 50 |

### Evaluation

The powder paint of the above composition was prepared. The surface tension value of the oily component of this paint was 2.95 x 10⁻² N/m. When this paint was used and applied to paper with friction, the paper could be painted.

### Powders A to C

Powder parts of the compositions shown in the following Table II-1 were prepared. Note that in Table II-1, the fluorine-treated talc (*) used was "PF-5 JA46R" (made by Daito Kasei). Further, the fluorine-treated sericite (**) used was "Eightpearl AL-300S) (made by Daito Kasei).

**Table II-1**

| Formulation ingredient | Powder A | Powder B | Powder C |
|---|---|---|---|
| Fluorine-treated talc (*) | 20 | 20 | - |
| Fluorine-treated sericite (**) | 80 | 70 | - |
| Silicic anhydride | - | - | 100 |
| Microparticulate titanium oxide | - | 10 | - |

### Water-in-Oil Emulsion Compositions (W/O) A to E

The water-in-oil emulsion compositions (W/O) A to E were prepared by the compositions shown in the following Table II-2. That is, the ingredients used among the ingredients (1) to (5) were mixed and dissolved (oil phase). On the other hand, the ingredients used from the ingredients (6) to (9) were separately mixed and dissolved (aqueous phase). Next, the aqueous phase was gradually added to the oil phase to prepare the emulsion.

**Table II-2**

| Formulation ingredient | W/O A | W/O B | W/O C | W/O D | W/O E |
|---|---|---|---|---|---|
| (1) Liquid paraffin | 60 | 55 | - | 10 | 36 |
| (2) Vaseline | 10 | 10 | - | 1.7 | - |
| (3) Dimethyl silicone | - | 5 | 70 | - | - |
| (4) Polyglyceryl diisostearate | 10 | 8 | - | 5 | 6 |
| (5) Dimeticon polyol | - | 2 | 10 | - | - |
| (6) Ion exchanged water | to 100 | to 100 | to 100 | to 100 | to 100 |
| (7) Sodium chloride | 1 | 1 | 1 | 1 | 1 |
| (8) 1,3-butylene glycol | 4 | 4 | 4 | 4 | 4 |
| (9) Preservative | q.s | q.s | q.s | q.s | q.s |
| Surface tension value (N/m) of outer phase (oil phase) | 2.84 x 10⁻² | 2.21 x 10⁻² | 1.9 x 10⁻² | 2.83 x 10⁻² | 3.0 x 10⁻² |

### Examples II-1 to II-6 and Comparative Examples II-1 to II-4

As shown in the following Tables II-3 and II-4, the powder parts shown in Table II-1 and the water-in-oil emulsion compositions shown in Table II-2 were mixed and stirred to prepare powder cosmetics. The above test methods were used to evaluate the feeling of use of Examples II-1 to II-6 and Comparative Examples II-1 to II-4 (lack of scratchy feeling, lack of sticky feeling, and fresh feeling) and the effect of suppression of adhesion of dirt to the container. The results are shown in Tables II-3 and II-4.

**Table II-3**

| Formulation ingredient | Ex. II-1 | Ex. II-2 | Ex. II-3 | Ex. II-4 | Ex. II-5 |
|---|---|---|---|---|---|
| Powder A | 35 | 35 | - | - | 70 |
| Powder B | - | - | 35 | 35 | - |
| Powder C | - | - | - | - | - |
| W/O A | 65 | - | 65 | - | 30 |
| W/O B | - | 65 | - | 65 | - |
| W/O C | - | - | - | - | - |
| W/O D | - | - | - | - | - |
| W/O E | - | - | - | - | - |
| Powderization | + | + | + | + | + |
| Lack of scratchy feeling | ++ | ++ | ++ | ++ | + |
| Lack of sticky feeling | | | | | |
| Fresh feeling | ++ | ++ | ++ | ++ | ++ |
| Effect of suppression of | + | + | + | + | + |
| adhesion of dirt to container | ++ | ++ | ++ | ++ | ++ |

**Table II-4**

| Formulation ingredient | Ex. II-6 | Comp. Ex. II-1 | Comp. Ex. II-2 | Comp. Ex. II-3 |
|---|---|---|---|---|
| Powder A | 35 | 35 | 35 | - |
| Powder B | - | - | - | - |
| Powder C | - | - | - | 35 |
| W/O A | - | - | - | 65 |
| W/O B | - | - | - | - |
| W/O C | - | 65 | - | - |
| W/O D | - | - | 65 | - |
| W/O E | 65 | - | - | - |
| Powderization | + | - | - | - |
| Lack of scratchy feeling | ++ | Not tested | Not tested | Not tested |
| Lack of sticky feeling Fresh feeling | ++ | Not tested | Not tested | Not tested |
| Effect of suppression | ++ | Not tested | Not tested | Not tested |
| of adhesion of dirt to container | ++ | Not tested | Not tested | Not tested |

As clear from the results of Tables II-3 and II-4, the product of each example could be powderized well and was superior in feeling of use (lack of scratchy feeling, lack of sticky feeling, fresh feeling). Further, no adhesion of dirt to the containers was seen. Note that as shown in Examples II-3 and II-4, it is also possible to blend in a non-fluorine-treated powder in addition to a fluorine-treated powder. Further, as explained above, if in a small amount so that the surface tension value of the oily component as a whole is maintained above 2.0 x 10⁻² N/m, the desired effect of the present invention can be obtained even if using an oil with a small surface tension value such as silicone oil or fluorocarbon oil.

### Example III-1 and Comparative Example III-1

A powder cosmetic (Example III-1) and oil cosmetic (Comparative Example III-1) were prepared by the compositions shown in the following Table III-1. The above test methods were used to evaluate the feeling of use (lack of scratchy feeling and lack of sticky feeling), the effect of suppression of chapping, and the effect of suppression of adhesion of dirt to the container of Example III-1 and Comparative Example III-1 and to measure the residual rate of the unstable ingredients. Note that in Table III-1, the fluorine-treated sericite (*) used was "PF-5 Eightpearl 300S-AL" (made by Daito Kasei). The results are shown in Table III-1.

**Table III-1**

| Formulation ingredient | Ex. III-1 | Comp. Ex. III-1 |
|---|---|---|
| (1) Fluorine-treated sericite (*) | 40 | - |
| (2) Pigment | 13 | - |
| (3) Macademia nut oil | 40 | 95 |
| (4) Vaseline | 6 | 4 |
| (5) Retinol | 0.5 | 0.5 |
| (6) L-ascorbic acid | 0.5 | 0.5 |
| Feeling in use (lack of scratchy feeling) | ++ | |
| Feeling in use (lack of sticky feeling) | ++ | - |
| Effect of suppression of chapping | ++ | ++ |
| Residual rate of retinol (50°C, 2 weeks, % by weight) | 27 | 10 |
| Residual rate of L-ascorbic acid (50°C, 2 weeks, % by weight) | 98 | 98 |
| Effect of suppression of adhesion of dirt to container | ++ | - |

### Production Method

In Example III-1, the ingredients (3) to (5) were heated to 50°C and mixed. The oil phase was then added to a powder of the ingredients (1), (2), and (6) homogeneously dispersed, then these mixed, stirred, and filled into a container. The surface tension value of the oily component of Example III-1 was 3.0 x 10⁻² N/m.

In Comparative Example III-1, the ingredients (3) to (5) were heated to 50°C and mixed. The oil phase was then added to a powder of the fine powder of ingredient (6) homogeneously dispersed, then these mixed, stirred, and filled into a container.

As clear from the results of Table III-1, the powder cosmetic of Example III-1 had an excellent feeling of use compared with the oil cosmetic of Comparative Example III-1 and was also superior in stability of the unstable medicine blended in (in particular the oil soluble medicine retinol). Further, no adhesion of dirt to the container was seen either.

### Example III-2: Whitener Powder

| | Formulation ingredients | % by weight |
|---|---|---|
| (1) | Fluorine-treated mica | 10 |
| (2) | Fluorine-treated sericite | 30 |
| (3) | Ganoderma powder | 0.1 |
| (4) | Albutin | 0.05 |
| (5) | Vaseline | 2.2 |
| (6) | Squalane | 8.6 |
| (7) | Pentaerythrityl tetraoctanoate | 47.3 |
| (8) | Beeswax | 1.1 |
| (9) | Retinol | 0.65 |

### Production Method

The homogeneously dissolved ingredients (5) to (9) were mixed in fine powder of the homogeneously dispersed ingredients (1), (2) and ingredients (3), (4) to prepare a powder cosmetic. The surface tension value of the oily component of Example II-2 was 2.6 x 10⁻² N/m.

The whitener powder of Example II-2 was superior in feeling of use (lack of scratchy feeling and lack of sticky feeling) and in effect in suppression of chapping and was superior in storage stability of the unstable medicinal ingredient. Further, adhesion of dirt to the container was also not seen.

### Example III-3: Moisture Retaining Powder

| | Formulation ingredients | % by weight |
|---|---|---|
| (1) | Fluorine-treated boron nitride | 5 |
| (2) | Fluorine-treated sericite | 30 |
| (3) | Hyaluronidase | 0.05 |
| (4) | Urea | 5 |
| (5) | Macademia nut oil | 20 |
| (6) | Liquid paraffin | 34.4 |
| (7) | β-carotin | 0.05 |
| (8) | Ceramide | 0.5 |
| (9) | Dynamite glycerin | 5 |

### Production Method

The homogeneously dissolved ingredients (5) to (9) were mixed in fine powder of the homogeneously dispersed ingredients (1), (2) and ingredients (3), (4) to prepare a powder cosmetic. The surface tension value of the oily component of Example III-3 was 3.0 x 10⁻² N/m.

The moisture retaining powder of Example III-3 was superior in feeling of use (lack of scratchy feeling and lack of sticky feeling) and in effect in suppression of chapping and was superior in storage stability of the unstable medicinal ingredient. Further, adhesion of dirt to the container was also not seen.

### Example III-4: Whitener and Sun Care Powder

| | Formulation ingredients | % by weight |
|---|---|---|
| (1) | Fluorine-treated kaolin | 5 |
| (2) | Fluorine-treated sericite | 30 |
| (3) | Microparticulate titanium oxide | 5 |
| (4) | Glycoside ascorbate | 3 |
| (5) | Glutathion | 0.1 |
| (6) | Octylmethoxycinnamate | 20 |
| (7) | Jojoba oil | 36.4 |
| (8) | Vitamin E acetate | 0.5 |

### Production Method

The homogeneously dissolved ingredients (6) to (8) were mixed in fine powder of the homogeneously dispersed ingredients (1) to (3) and ingredients (4), (5) to prepare a powder cosmetic. The surface tension value of the oily component of Example III-4 was 3.3 x 10⁻² N/m.

The whitener and sun care powder of Example III-4 was superior in feeling of use (lack of scratchy feeling and lack of sticky feeling) and in effect in suppression of chapping and was superior in storage stability of the unstable medicinal ingredients. Further, adhesion of dirt to the container was also not seen.

### Example III-5: Athletes Foot Powder

| | Formulation ingredients | % by weight |
|---|---|---|
| (1) | Fluorine-treated kaolin | 10 |
| (2) | Fluorine-treated sericite | 33 |
| (3) | Benzalkonium chloride | 3 |
| (4) | Vaseline | 3 |
| (5) | Macademia nut oil | 50 |
| (6) | Dimethyl silicone | 0.2 |
| (7) | Vitamin E acetate | 0.2 |
| (8) | Silver zeolite | 0.6 |

### Production Method

The homogeneously dissolved ingredients (4) to (7) were mixed in fine powder of the homogeneously dispersed ingredients (1), (2) and ingredients (3), (8) to prepare a powder cosmetic. The surface tension value of the oily component of Example III-5 was 3.0 x 10⁻² N/m.

The athlete's foot powder of Example III-5 was superior in feeling of use (lack of scratchy feeling and lack of sticky feeling) and in effect in suppression of chapping and was superior in storage stability of the unstable medicinal ingredients. Further, adhesion of dirt to the container was also not seen.

### INDUSTRIAL APPLICABILITY

The powder composition of the present invention liquifies upon the friction of an application thereof at the time of use in spite of a powder in form, does not give a sticky feeling during use, can suppress chapping of the skin, can effectively prevent adhesion of oil, dirt, etc. to the container in spite of an oil-rich preparation, can impart a fresh feeling, is superior in impact stability, and can be widely used as a new type of powder composition. Further, according to the present invention, a new type of powder cosmetic composition is provided which, while an oil-rich preparation highly effective in suppressing chapping of the skin, is superior in feeling of use having no stickiness when applied to the skin, is easy to use with no oil adhering to the storage container even with continuous long term use, stably includes unstable ingredients which would easily break down, spoil, etc. in the presence of light, oxygen, water etc., and can sufficiently exhibit the functions of the same. The powder composition of the present invention is particularly suitably used as a powder cosmetic composition, powder paint, etc.

## Claims

1. A powder cosmetic composition comprising
(a) a fluorine-treated powder and
(b) an oily component,
without substantially containing water,
wherein
component (a) is present in an amount of 20 to 70% by weight based upon the total amount of the composition,
the oily component (b) has a surface tension value (average) with respect to the atmosphere of at least 2.0 x 10⁻² N/m,
the ratio of the component (a) and component (b) is 1:9 to 4:1 (ratio by weight)
and
the oily component (b) is powderized by absorbing component (a),
which composition is capable of liquifying by friction on application thereof.

2. A powder cosmetic composition as claimed in claim 1, wherein the component (a) is a fluorine-treated powder of a clay mineral treated on its surface with fluorine.

3. A powder cosmetic composition as claimed in claim 1, wherein the component (a) is a fluorine-treated powder of a clay mineral treated on its surface with at least one gel selected from the group consisting of hydrates, partial dehydrates, and anhydrides of metal hydroxides and metal salts, followed by being treated with fluorine.

4. A powder cosmetic composition as claimed in claim 2 or 3, wherein said clay mineral is at least one clay mineral selected from the group consisting of talc, kaolin, silicic anhydride, saponite, sericite, mica and boron nitride.

5. A powder cosmetic composition as claimed in any one of claims 1 to 4, **characterized by** further comprising (c) a component unstable in the presence of light, oxygen or water.

6. A powder cosmetic composition as claimed in claim 5, wherein the component (a) is a fluorine-treated powder of a clay mineral treated on its surface with fluorine.

7. A powder cosmetic composition as claimed in claim 6, wherein the component (c) is at least one member selected from the group consisting of whitener, anti- inflammatory agent, humectant, blood circulation promoter, hair tonic, antibacterial agent, hormone, vitamin, enzyme, antioxidant and animal or plant extract.

8. A powder cosmetic composition as claimed in any one of claims 5 to 7, comprising the component (c) in an amount of 0.001 to 10% by weight.

9. A powder cosmetic composition as claimed in any one of claims 1 to 8, **characterized in that** the fluorine- treated powder is a powder treated on part or all of the powder surface with a perfluoroalkyl phosphoric acid ester diethanolamine salt-having the formula (I): 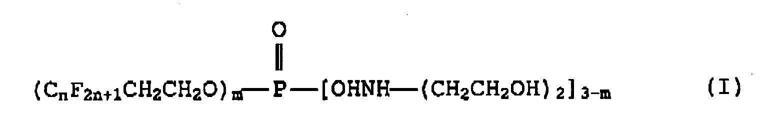 wherein n is an integer of 5 to 20 and m is 1 or 2.

10. A powder cosmetic composition as claimed in any one of claims 1 to 8, wherein the fluorine-treated powder is a powder treated on part or all of the powder surface with a perfluoroalkylsilane having the formula (II):
CₐF₂ₐ₊₁-(CH₂)_{b}-SiX₃ (II)
wherein a is an integer of 1 to 12, b is an integer of 1 to 5 and X is a halogen atom, an alkyl group or an alkoxy group.

11. A powder cosmetic composition as claimed in any one of claims 1 to 8, wherein the fluorine-treated powder is a powder treated on part or all of the powder surface with a perfluoroalkylethyl acrylate having the formula (III): 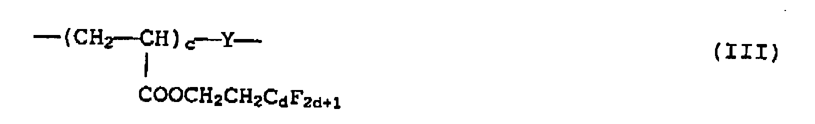 wherein c is an integer of 1 to 12, d is an integer of 5 to 20 and Y is polyethylene glycol or an alkyl copolymer containing a silicone chain or an acryl group.

## Patentansprüche

1. Kosmetische Puderzusammensetzung, umfassend
(a) ein fluorbehandeltes Pulver und
(b) eine ölige Komponente,
ohne wesentlich Wasser zu enthalten,
wobei
die Komponente (a) in einer Menge von 20 bis 70 Gew.-%, bezogen auf die Gesamtmenge der Zusammensetzung, vorhanden ist,
die ölige Komponente (b) einen Oberflächenspannungswert (Mittelwert) in Bezug auf die Atmosphäre von mindestens 2,0 x 10⁻² N/m aufweist,
das Verhältnis der Komponente (a) und der Komponente (b) 1:9 bis 4:1 (Gewichtsverhältnis) beträgt und
die ölige Komponente (b) durch Absorbieren der Komponente (a) in den Pulverzustand überführt wird,
wobei die Zusammensetzung beim Auftragen derselben durch Reibung flüssig werden kann.

2. Kosmetische Puderzusammensetzung nach Anspruch 1, wobei die Komponente (a) ein fluorbehandeltes Pulver eines Tonminerals, das auf dessen Oberfläche mit Fluor behandelt wurde, ist.

3. Kosmetische Puderzusammensetzung nach Anspruch 1, wobei die Komponente (a) ein fluorbehandeltes Pulver eines Tonminerals ist, das auf dessen Oberfläche mit mindestens einem Gel, das aus der Gruppe von Hydraten, partiellen Dehydraten und Anhydriden von Metallhydroxiden und Metallsalzen ausgewählt ist, behandelt wurde und anschließend mit Fluor behandelt wurde.

4. Kosmetische Puderzusammensetzung nach Anspruch 2 oder 3, wobei das Tonmineral mindestens ein Tonmineral ist, das aus der Gruppe von Talkum, Kaolin, Kieselsäureanhydrid, Saponit, Sericit, Glimmer und Bornitrid ausgewählt ist.

5. Kosmetische Puderzusammensetzung nach einem der Ansprüche 1 bis 4, **dadurch gekennzeichnet, dass** sie ferner (c) eine in Gegenwart von Licht, Sauerstoff oder Wasser instabile Komponente umfasst.

6. Kosmetische Puderzusammensetzung nach Anspruch 5, wobei die Komponente (a) ein fluorbehandeltes Pulver eines Tonminerals, das auf dessen Oberfläche mit Fluor behandelt wurde, ist.

7. Kosmetische Puderzusammensetzung nach Anspruch 6, wobei die Komponente (c) mindestens eine Komponente ist, die aus der Gruppe von einem Weißmacher, entzündungshemmenden Mittel, Feuchthaltemittel, durchblutungsfördernden Mittel, Haarpflegemittel, antibakteriellen Mittel, Hormon, Vitamin, Enzym, Antioxidationsmittel und tierischen oder pflanzlichen Extrakt ausgewählt ist.

8. Kosmetische Puderzusammensetzung nach einem der Ansprüche 5 bis 7, die die Komponente (c) in einer Menge von 0,001 bis 10 Gew.-% umfasst.

9. Kosmetische Puderzusammensetzung nach einem der Ansprüche 1 bis 8, **dadurch gekennzeichnet, dass** das fluorbehandelte Pulver ein Pulver ist, das auf einem Teil der oder der gesamten Pulveroberfläche mit einem Perfluoralkylphosphorsäureester-Diethanolamin-Salz der Formel (I) : 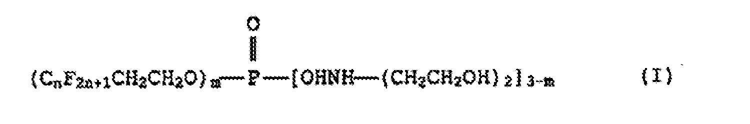 worin n für eine ganze Zahl von 5 bis 20 steht und m für 1 oder 2 steht,
behandelt wurde.

10. Kosmetische Puderzusammensetzung nach einem der Ansprüche 1 bis 8, wobei das fluorbehandelte Pulver ein Pulver ist, das auf einem Teil der oder der gesamten Pulveroberfläche mit einem Perfluoralkylsilan der Formel (II):
CₐF₂ₐ₊₁-(CH₂)_{b}-SiX₃ (II)
worin a für eine ganze Zahl von 1 bis 12 steht, b für eine ganze Zahl von 1 bis 5 steht und X für ein Halogenatom, eine Alkylgruppe oder eine Alkoxygruppe steht, behandelt wurde.

11. Kosmetische Puderzusammensetzung nach einem der Ansprüche 1 bis 8, wobei das fluorbehandelte Pulver ein Pulver ist, das auf einem Teil der oder der gesamten Pulveroberfläche mit einem Perfluoralkylethylacrylat der Formel (III): 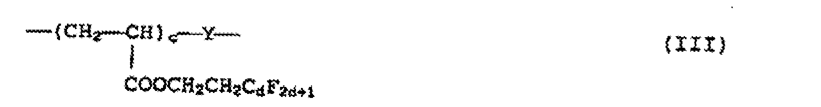 worin c für eine ganze Zahl von 1 bis 12 steht, d für eine ganze Zahl von 5 bis 20 steht und Y für ein Polyethylenglykol oder ein eine Siliconkette oder eine Acrylgruppe enthaltendes Alkylcopolymer steht,
behandelt wurde.

## Revendications

1. Composition cosmétique en poudre comprenant
(a) une poudre traitée avec du fluor et
(b) un constituant huileux,
sans contenir substantiellement de l'eau,
dans laquelle
le constituant (a) est présent en une quantité de 20 à 70 % en poids sur la base de la quantité totale de la composition,
le constituant huileux (b) a une valeur (moyenne) de tension superficielle par rapport à l'atmosphère d'au moins 2,0 x 10⁻² N/m,
le rapport du constituant (a) et du constituant (b) va de 1:9 à 4:1 (rapport en poids)
et
le constituant huileux (b) est mis sous forme de poudre par absorption du constituant (a),
composition qui peut subir une liquéfaction par frottement lors de l'application.

2. Composition cosmétique en poudre suivant la revendication 1, dans laquelle le constituant (a) est une poudre traitée avec du fluor constituée d'une substance minérale argileuse traitée sur sa surface avec du fluor.

3. Composition cosmétique en poudre suivant la revendication 1, dans laquelle le constituant (a) est une poudre traitée avec du fluor constituée d'une substance minérale argileuse traitée sur sa surface avec au moins un gel choisi dans le groupe consistant en des hydrates, des produits de déshydratation partielle, et des anhydrides d'hydroxydes métalliques et sels métalliques, puis traitée avec du fluor.

4. Composition cosmétique en poudre suivant la revendication 2 ou 3, dans laquelle ladite substance minérale argileuse est au moins une substance minérale argileuse choisie dans le groupe consistant en le talc, le kaolin, l'anhydride silicique, la saponite, la séricite, le mica et le nitrure de bore.

5. Composition cosmétique en poudre suivant l'une quelconque des revendications 1 à 4, **caractérisée en ce qu'**elle comprend en outre (c) un constituant instable en présence de lumière, d'oxygène ou d'eau.

6. Composition cosmétique en poudre suivant la revendication 5, dans laquelle le constituant (a) est une poudre traitée avec du fluor constituée d'une substance minérale argileuse traitée sur sa surface avec du fluor.

7. Composition cosmétique en poudre suivant la revendication 6, dans laquelle le constituant (c) est au moins un membre choisi dans le groupe consistant en un agent de blanchiment, un agent anti-inflammatoire, un agent humidifiant, un agent activant la circulation sanguine, un tonique capillaire, un agent antibactérien, une hormone, une vitamine, une enzyme, un antioxydant et un extrait animal ou végétal.

8. Composition cosmétique en poudre suivant l'une quelconque des revendications 5 à 7, comprenant le constituant (c) en une quantité de 0,001 à 10 % en poids.

9. Composition cosmétique en poudre suivant l'une quelconque des revendications 1 à 8, **caractérisée en ce que** la poudre traitée avec du fluor est une poudre traitée sur une partie ou sur la totalité de la surface de la poudre avec un sel de diéthanolamine d'ester d'acide perfluoralkylphosphorique répondant à la formule (I) : 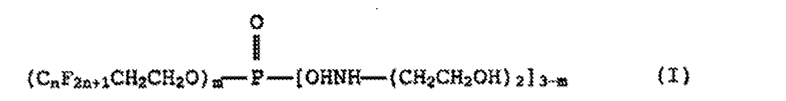 dans laquelle n représente un nombre entier de 5 à 20 et m est égal à 1 ou 2.

10. Composition cosmétique en poudre suivant l'une quelconque des revendications 1 à 8, dans laquelle la poudre traitée avec du fluor est une poudre traitée sur une partie ou sur la totalité de la surface de la poudre avec un perfluoralkylsilane, répondant à la formule (II) :
CₐF₂ₐ₊₁-(CH₂)_{b}-SiX₃ (II)
dans laquelle a représente un nombre entier de 1 à 12, b représente un nombre entier de 1 à 5 et X représente un atome d'halogène, un groupe alkyle ou un groupe alkoxy.

11. Composition cosmétique en poudre suivant l'une quelconque des revendications 1 à 8, dans laquelle la poudre traitée avec du fluor est une poudre traitée sur une partie ou la sur totalité de la surface de la poudre avec un acrylate perfluoralkyléthylique répondant à la formule (III) : 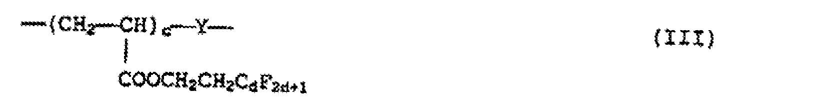 dans laquelle c représente un nombre entier de 1 à 12, d représente un nombre entier de 5 à 20 et Y représente un polyéthylèneglycol ou un copolymère alkylique contenant une chaîne silicone ou un groupe acryle.
